# EUROPEAN PATENT SPECIFICATION

(11) **EP 2 421 539 B1**
(45) Date of publication and mention of the grant of the patent: **23.10.2019**
(21) Application number: 10767800.5
(22) Date of filing: 22.04.2010
(51) Int. Cl.: A61K 31/538, A61K 31/47, A61P 31/00, A61K 9/12, A61P 11/00

(54) **METHODS OF TREATING A PULMONARY BACTERIAL INFECTION USING FLUORO-QUINOLONES**
VERFAHREN ZUR BEHANDLUNG EINER BAKTERIELLEN INFEKTION DER LUNGE MITHILFE VON FLUORCHINOLONEN
MÉTHODES DE TRAITEMENT D'UNE INFECTION PULMONAIRE BACTÉRIENNE UTILISANT DES FLUOROQUINOLONES

(30) Priority: 24.04.2009 US 172625 P
(43) Date of publication of application: 29.02.2012
(60) Divisional application: 18189712.5
(73) Proprietor: Horizon Orphan LLC, Lake Forest, IL 60045 (US)
(72) Inventor: DUDLEY, Mike, San Diego CA 92127 (US); GRIFFITH, David, San Marcos CA 92069 (US); RODNY, Olga, Mountain View CA 94040 (US)
(74) Representative: HGF Limited
(86) International application number: PCT/US2010/032128
(87) International publication number: WO 2010/124141

(56) References cited:
- WO-A1-2009/140587
- WO-A1-2010/042553
- WO-A2-2006/125132
- US-A1- 2004 009 126
- PALMER, K.L. ET AL.: 'Membrane-Bound Nitrate Reductase Is Required for Anaerobic Growth in Cystic Fibrosis Sputum' JOURNAL OF BACTERIOLOGY. vol. 189, no. 12, 2007, pages 4449 - 4455, XP055093677
- JENSEN, T. ET AL.: 'The Efficacy and Safety of Ciprofloxacin and Ofloxacin in Chronic Pseudomonas aeruginosa Infection in Cystic Fibrosis'' JOURNAL OF ANTIMICROBIAL CHEMOTHERAPY. vol. 20, no. 4, 1987, pages 585 - 594, XP009175210
- LEE, C.K.K. ET AL.: 'Levofloxacin Pharmacokinetics in Adult Cystic Fibrosis' CHEST. vol. 131, no. 3, 2007, pages 796 - 802, XP055094289
- KING, P. ET AL.: 'Effect of Oxygen Limitation on the In Vitro Activity of Levofloxacin and Other Antibiotics Administered by the Aerosol Route Against Pseudomonas aeruginosa from Cystic Fibrosis Patients' DIAGNOSTIC MICROBIOLOGY AND INFECTIOUS DISEASE. vol. 66, no. 2, 2010, pages 181 - 186, XP026851080

## Description

### CROSS-REFERENCE TO RELATED APPLICATIONS

This application claims the benefit of priority to U.S. Provisional Application No. 61/172,625, filed April 24, 2009.

### BACKGROUND

### Field

This application relates to the fields of pharmaceutical chemistry and medicine. In particular, it relates to levofloxacin for use in methods of treating pulmonary bacterial infections.

### Description of the Related Art

The pathogen associated with most chronic infections affecting cystic fibrosis (CF) patients is *Pseudomonas aeruginosa.* According to the Cystic Fibrosis Foundation (CFF), approximately 55% of CF patients are colonized with *P. aeruginosa.* Severe pulmonary exacerbations are a common manifestation from chronic *P. aeruginosa* infections.

*P. aeruginosa* can grow under anaerobic conditions by using nitrate or nitrite for anaerobic respiration, or by fermentation of arginine. Sputum from CF patients contain average nitrate levels of 250-350 µM and can contain levels as high as 1000 µM. Therefore, CF sputum can provide *P. aeruginosa* cells with an environment in which to promote and sustain colonization under anaerobic conditions.

It has been demonstrated that areas of low oxygen tension exist within dense pulmonary secretions in the lungs of CF patients. Although typically aerobic, *P. aeruginosa* can colonize and proliferate within these microaerophilic environments in CF sputum.

WO 2006/125132 A2 relates to formulations of fluoroquinolones suitable for aerosolization and use of such formulations for aerosol administration of fluoroquinolone antimicrobials for the treatment of pulmonary bacterial infections. In particular, inhaled levofloxacin specifically formulated and delivered for bacterial infections of the lungs is described.

US 2004/009126 A1 relates to the treatment of pulmonary infections by administering antibacterial agents or antiviral agents by inhalation.

Jensen, T. et al, "The efficacy and safety of ciprofloxacin and ofloxacin in chronic Pseudomonas aeruginosa infection in cystic fibrosis", Journal of Antimicrobial Chemotherapy, vol 20, No. 4, 1987, pages 585-594, suggests using ciprofloxacin and ofloxacin as agents for intermittent treatment of chronic P. aeruginosa lung infection in adult cystic fibrosis patients.

Lee, C.K.K. et al, "Levofloxacin pharmacokinetics in adult cystic fibrosis, Chest., vol 131, No 3, 2007, pages 796-802 discuss the pharmacokinetics of levofloxacin in adult cystic fibrosis patients.

WO 2009/140587 A1 relates to a composition for pulmonary administration comprising a fluoroquinolone betaine, such as ciprofloxacin betaine. The composition is asserted to be useful in treating an endobronchial infection, such as Pseudomonas aeruginosa, and is asserted to be particularly useful in treating cystic fibrosis.

WO 2010/042553 relates to the use of aerosolized fluoroquinolones formulated with divalent or trivalent cations for the treatment and management of bacterial infections of the lung and upper respiratory tract.

King, P. et al, "Effect of oxygen limitation on the in vitro activity of levofloxacin and other antibiotics administered by the aerosol route against Pseudomonas aeruginosa from cystic fibrosis patients", Diagnostic Microbiology and Infectious Disease, vol 66, No 2, 2010, pages 181-186, discuss the activity of levofloxacin against P. aeruginosa under anaerobic or hypoxic conditions.

### SUMMARY

In a first aspect, the invention relates to a fluoroquinolone antibiotic for use in a method of treating a pulmonary bacterial infection including administering a therapeutically effective amount of an aerosol of the fluoroquinolone antibiotic, wherein the fluoroquinolone antibiotic is levofloxacin, wherein the pulmonary bacterial infection includes *Pseudomonas aeruginosa* bacteria capable of growing under anaerobic conditions, and wherein the bacteria is exposed to at least 0.75 mg/L of the fluoroquinolone antibiotic.

In some embodiments, the method includes assaying the pulmonary bacterial infection for the presence of bacteria growing under anaerobic conditions. The bacteria, in some embodiments, are growing under anaerobic conditions using nitrate or nitrite.

Some embodiments further include assaying the pulmonary bacterial infection for the presence of bacteria growing under anaerobic conditions using nitrate or nitrite. In some embodiments, the method includes assaying the pulmonary bacterial infection for the presence of Pseudomonas aeruginosa.

In some embodiments, at least a portion of the pulmonary bacterial infection is growing under anaerobic conditions. In some embodiments, the pulmonary bacterial infection is identified as having at least a portion of said bacteria growing under anaerobic conditions.

Some embodiments have the pulmonary bacterial infection in a subject with cystic fibrosis. Some embodiments have the pulmonary infection characterized by sputum including nitrate levels of at least 250 µM. In some embodiments, pulmonary bacterial infection is identified as having sputum comprising nitrate levels of at least 250 µm.

In some embodiments, the method of treating the pulmonary bacterial infection does not include administering a therapeutically effective amount of an antibiotic selected from the group consisting of tobramycin, amikacin and aztreonam.

In some embodiments, no other antibiotics are administered in a therapeutically effective amount to treat the pulmonary bacterial infection. In some embodiments, the fluoroquinolone antibiotic is administered by intrapulmonary delivery. In some embodiments, the therapeutically effective amount of fluoroquinolone is more than about 5 mg. In some embodiments, the therapeutically effective amount of fluoroquinolone os no more than about 150 mg.

In a second aspect, the invention relates to a fluoroquinolone antibiotic for use in a method of inhibiting *Pseudomonas aeruginosa* bacteria growing under anaerobic conditions comprising exposing said bacteria to an amount of a fluoroquinolone antibiotic effective to inhibit the growth of said bacteria, wherein the bacteria is exposed to a mixture comprising at least 0.75 mg/L of the fluoroquinolone antibiotic, wherein the fluoroquinolone antibiotic is levofloxacin.

Some embodiments of the method include assaying a sample of said bacteria to determine if the bacteria is growing under anaerobic conditions. In some embodiments, a sample of the bacteria is characterized by nitrate levels of at least 250 µm.

In a third aspect, the invention relates to a fluoroquinolone antibiotic for use in a method of inhibiting *Pseudomonas aeruginosa* bacteria growing under anaerobic conditions comprising exposing said bacteria to an effective amount of a fluoroquinolone antibiotic to inhibit the growth of said bacteria, wherein the fluoroquinolone antibiotic is levofloxacin at a concentration ranging from 0.125 mg/L to 128 mg/L.

### BRIEF DESCRIPTION OF THE DRAWINGS

**FIG. 1** is a table showing aerobic and anaerobic MIC testing of various antimicrobials.
**FIG. 2A** is a graph of the *P. aeruginosa* aerobic and anaerobic MIC distributions of levofloxacin (LVX).
**FIG. 2B** is a graph of the *P. aeruginosa* aerobic and anaerobic MIC distributions of tobramycin (TOB).
**FIG. 2C** is a graph of the *P. aeruginosa* aerobic and anaerobic MIC distributions of amikacin (AMK).
**FIG. 2D** is a graph of the *P. aeruginosa* aerobic and anaerobic MIC distributions of aztreonam (ATM).
**FIG. 3A** is a graph of the mean log CFU/mL of *P. aeruginosa* over time for the strain PAM1020, wild-type.
**FIG. 3B** is a graph of the mean log CFU/mL of *P. aeruginosa* over time for the strain PAM1032, *na1B.*
**FIG. 3C** is a graph of the mean log CFU/mL of *P. aeruginosa* over time for the strain PAM1481, *na1B gyrA.*
**FIG. 3D** is a graph of the mean log CFU/mL of *P. aeruginosa* over time for the strain PAM1573, *na1B gyrA* (Thr83Ile).

### DETAILED DESCRIPTION OF THE PREFERRED EMBODIMENT

Cystic fibrosis is a hereditary disease that results in frequent pulmonary bacterial infections requiring treatment with antibiotics. U.S. Publication No. 2006/0276483 teaches aerosolized fluoroquinolones and their uses for treating bacterial pulmonary infections.

Some types of bacteria that may be present in a pulmonary bacterial infection can grow under anaerobic conditions. It has been demonstrated that areas of low oxygen tension exist within dense pulmonary secretions in the lungs of CF patients. Thus, a pulmonary bacterial infection may have bacteria growing under anaerobic conditions. Hypoxic environments, which can be found in CF patients, can impede the potency of some classes of antibiotics and therefore improved methods of treatment are necessary.

Surprisingly, it has been found that fluoroquinolones exhibit similar activity against bacteria growing in both aerobic and anaerobic conditions.

### Definitions

The term "microbe" refers to microscopic organisms, such bacteria or fungi. Thus, any disclosure of this term also contemplates features relating to the narrower class of "bacteria." For example, descriptions relating to antimicrobial compounds also contemplate using antibiotics.

The term "administration" or "administering" refers to a method of giving a dosage of an antimicrobial pharmaceutical composition to a vertebrate. The preferred method of administration can vary depending on various factors, e.g., the components of the pharmaceutical composition, the site of the potential or actual bacterial infection, the microbe involved, and the severity of an actual microbial infection.

The term "mammal" is used in its usual biological sense. Thus, it specifically includes humans, cattle, horses, dogs, and cats, but also includes many other species.

The term "microbial infection" refers to the undesired proliferation or presence of invasion of pathogenic microbes in a host organism. This includes the excessive growth of microbes that are normally present in or on the body of a mammal or other organism. More generally, a microbial infection can be any situation in which the presence of a microbial population(s) is damaging to a host mammal. Thus, a microbial infection exists when excessive numbers of a microbial population are present in or on a mammal's body, or when the effects of the presence of a microbial population(s) is damaging the cells or other tissue of a mammal.

In the context of the response of a microbe, such as a bacterium, to an antimicrobial agent, the term "susceptibility" refers to the sensitivity of the microbe for the presence of the antimicrobial agent. So, to increase the susceptibility means that the microbe will be inhibited by a lower concentration of the antimicrobial agent in the medium surrounding the microbial cells. This is equivalent to saying that the microbe is more sensitive to the antimicrobial agent. In most cases the minimum inhibitory concentration (MIC) of that antimicrobial agent will have been reduced.

By "therapeutically effective amount" or "pharmaceutically effective amount" is meant an amount of fluoroquinolone antimicrobial agent, which has a therapeutic effect. The doses of fluoroquinolone antimicrobial agent which are useful in treatment are therapeutically effective amounts. Thus, as used herein, a therapeutically effective amount means those amounts of fluoroquinolone antimicrobial agent which produce the desired therapeutic effect as judged by clinical trial results and/or model animal infection studies. In particular embodiments, the fluoroquinolone antimicrobial agent are administered in a pre-determined dose, and thus a therapeutically effective amount would be an amount of the dose administered. This amount and the amount of the fluoroquinolone antimicrobial agent can be routinely determined by one of skill in the art, and will vary, depending on several factors, such as the particular microbial strain involved. This amount can further depend upon the patient's height, weight, sex, age and medical history. For prophylactic treatments, a therapeutically effective amount is that amount which would be effective to prevent a microbial infection.

A "therapeutic effect" relieves, to some extent, one or more of the symptoms of the infection, and includes curing an infection. "Curing" means that the symptoms of active infection are eliminated, including the total or substantial elimination of excessive members of viable microbe of those involved in the infection to a point at or below the threshold of detection by traditional measurements. However, certain long-term or permanent effects of the infection may exist even after a cure is obtained (such as extensive tissue damage). As used herein, a "therapeutic effect" is defined as a statistically significant reduction in bacterial load in a host, emergence of resistance, or improvement in infection symptoms as measured by human clinical results or animal studies.

"Treat," "treatment," or "treating," as used herein refers to administering a pharmaceutical composition for prophylactic and/or therapeutic purposes. The term "prophylactic treatment" refers to treating a patient who is not yet infected, but who is susceptible to, or otherwise at risk of, a particular infection. The term "therapeutic treatment" refers to administering treatment to a patient already suffering from an infection. Thus, in preferred embodiments, treating is the administration to a mammal (either for therapeutic or prophylactic purposes) of therapeutically effective amounts of a fluoroquinolone antimicrobial agent.

### Levofloxacin for use in a method of Treatment

In a first aspect, the invention relates to a fluoroquinolone antibiotic for use in a method of treating a pulmonary bacterial infection that include administering a therapeutically effective amount of an aerosol of the fluoroquinolone antimicrobial, wherein the fluoroquinolone antibiotic is levofloxacin, wherein the pulmonary bacterial infection comprises *Pseudomonas aeruginosa* bacteria growing under anaerobic conditions and wherein the bacteria is exposed to at least 0.75 mg/L of the fluoroquinolone antibiotic.

The therapeutically effective amount may include, for example, at least about 5 mg; at least about 10 mg; at least about 20 mg; or at least about 50 mg. Similarly, therapeutically effective amount may include, for example, no more than about 150 mg; no more than about 140 mg; no more than about 125 mg; or no more than about 100 mg.

Because fluoroquinolones exhibit activity against bacteria growing under anaerobic conditions, the method may include assaying the pulmonary bacteria infection for the presence of bacteria growing, or capable of growing, under anaerobic conditions. For example, a culture may be taken of the infection and the type of bacteria present determined. If there are bacteria capable of growing under anaerobic conditions, a treatment including administering a fluoroquinolone can be used. Moreover, using such an assay, other criteria may be used to determine if a treatment including administering a fluoroquinolone is appropriate. A fluoroquinolone may be appropriate when there are bacteria growing under anaerobic condition using a nitrite or nitrate, or alternatively, when the bacteria is *Pseudomonas aeruginosa.*

The fluoroquinolone is levofloxacin. The levofloxacin is in aerosol form to allow intrapulmonary delivery.

In some embodiments, the method does not include treating the pulmonary bacterial infection with a therapeutically effective amount of tobramycin, amikacin or aztreonam. In another embodiment, no other antimicrobials are administered in a therapeutically effective amount to treat the pulmonary bacterial infection.

The type of pulmonary infections to be treated is not particularly limited. The pulmonary infection may include an infection found in a patient with cystic fibrosis. Also, the method may be used to treat a pulmonary bacterial infection that is characterized by sputum comprising average nitrate levels of at least about 250 µM or at least about 500 µM. Finally, the method may be used for a pulmonary bacterial infection that has at least a portion of the bacteria growing under anaerobic conditions.

In an embodiment, the treatment includes *Pseudomonas aeruginosa* bacteria growing, or capable of growing, under anaerobic conditions using nitrate or nitrite.

### EXAMPLES

Embodiments of the present application are disclosed in further detail in the following examples, which are not in any way intended to limit the scope of the invention. Only the examples relating to exposing *Pseudomonas aeruginosa* to levofloxacin are embodiments of the present invention as defined in the claims.

### Bacterial strains and antibiotics

One hundred and fourteen CF *P. aeruginosa* isolates were obtained for susceptibility testing from the CF Referral Center for Susceptibility & Synergy Studies at Columbia University (New York, NY) and also from two CF Therapeutics Development Network (TDN) laboratories (Seattle Children's Hospital, Seattle, WA and University of North Carolina at Chapel Hill, Chapel Hill, NC). About sixty percent were recent isolates (2004-2007) with the remaining forty percent isolated between 1980 and 2004.

*P. aeruginosa* strains PAM1020 (wild-type), PAM1032 (*na1B*), PAM1481 (*na1B gyrA* (*Asp87Tyr*))*,* and PAM1573 (*na1B gyrA* (Thr83Ile)) represent relevant efflux-mediated and target mutation resistance mechanisms and were used in the levofloxacin time-kill assays.

The antibiotics used in these studies included tobramycin, levofloxacin, amikacin, and aztreonam which are in use or in development as aerosolized therapies for CF. For aerobic susceptibility tests, levofloxacin hydrochloride, tobramycin sulfate, and amikacin disulfate were purchased from LKT Laboratories (St. Paul, MN) and aztreonam base was purchased from MP Biomedicals (Solon, OH). All antibiotics used for anaerobic susceptibility tests were purchased from the United States Pharmacopeia (Rockville, MD).

### Susceptibility testing

Antibiotic MIC endpoints were obtained using the broth microdilution method according to the CLSI reference method. See Clinical and Laboratory Standards Institute. Methods for Dilution Antimicrobial Susceptibility Tests for Bacteria That Grow Aerobically - Seventh Edition: Approved Standard M7-A7. CLSI, Wayne, PA, USA, 2006. Antibiotics were serially diluted to the following concentrations for aerobic testing: levofloxacin and tobramycin from 0.03 - 32 mg/L and amikacin and aztreonam from 0.125 - 128 mg/L. Anaerobic susceptibility testing required the addition of 1% potassium nitrate (KNO₃) to cation-adjusted Mueller-Hinton broth (CAMHB) to allow for *P*. *aeruginosa* anaerobic respiration. For anaerobic testing, frozen MIC plates were thawed and stored in the anaerobic chamber overnight to ensure elimination of all oxygen prior to inoculation with test strains. The dilution range for all antibiotics was 0.125 - 128 mg/L for the anaerobic susceptibility tests. Extended incubation of up to 48 hours under anaerobic conditions may be required and was needed for 54% of the isolates.

### Bactericidal activity

Aerobic and hypoxic time-kill assays were conducted to determine the bactericidal activity of levofloxacin at concentrations ranging from 32 - 1,024 mg/L. Levofloxacin concentrations ranged from 16-fold to 2,048-fold the MIC against isogenic *P*. *aeruginosa* strains PAM1020 (MIC = 0.125 mg/L), PAM1032 (MIC =1 mg/L), PAM1481 (MIC = 4 mg/L) and PAM1573 (MIC = 8 mg/L). Aerobic and hypoxic Mueller-Hinton broth (MHB) cultures were diluted to an initial inoculum of 1 × 10⁷ - 1 × 10⁸ CFU/ml. Hypoxic conditions were simulated by maximizing the MHB volume in the growth vessel and omitting shaking during incubation at 37°C. Growth rates using these conditions or MHB treated with the Oxyrase enzyme system (Oxyrase, Inc., Mansfield, OH) were similar. The final culture volume was 10 ml. At 0, 10, 20, 40, 80 and 160 minutes, 0.5 ml samples were removed from each culture, immediately washed twice with MHB to minimize levofloxacin carryover effects, serially diluted with physiologic saline and plated on Mueller-Hinton agar (MHA). Agar plates were incubated up to 48 hours at 37°C and bactericidal activity was assessed. The limit of detection was 2 login CFU/ml. Bacterial counts obtained following incubation under either condition were compared using a paired t-test.

### Results

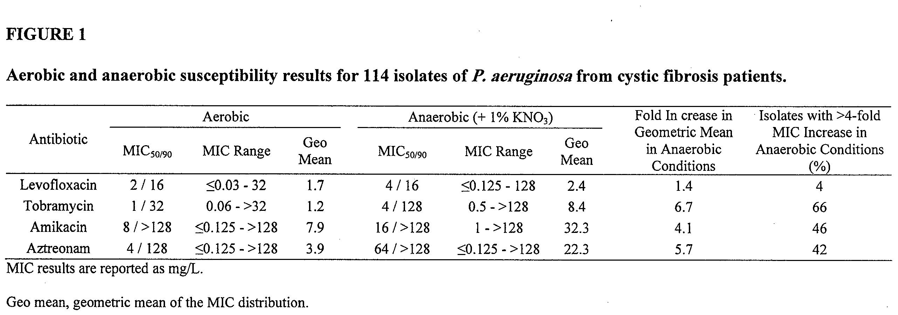

The results for aerobic and anaerobic MIC testing are summarized in the table shown in FIG. 1. There was little change in the potency of levofloxacin under anaerobic conditions; the MIC₅₀ only increased 2-fold, with no increase in MIC₉₀. In contrast, anaerobic incubation increased the geometric means of the MIC for tobramycin, amikacin, and aztreonam by approximately 7-fold, 4-fold, and 6-fold, respectively, with MIC₅₀ values for tobramycin and aztreonam increasing 4- and 16-fold, respectively under anaerobic conditions. More than 40% of the isolates had MICs increase >4-fold to tobramycin, amikacin and aztreonam compared to only 4% for levofloxacin.

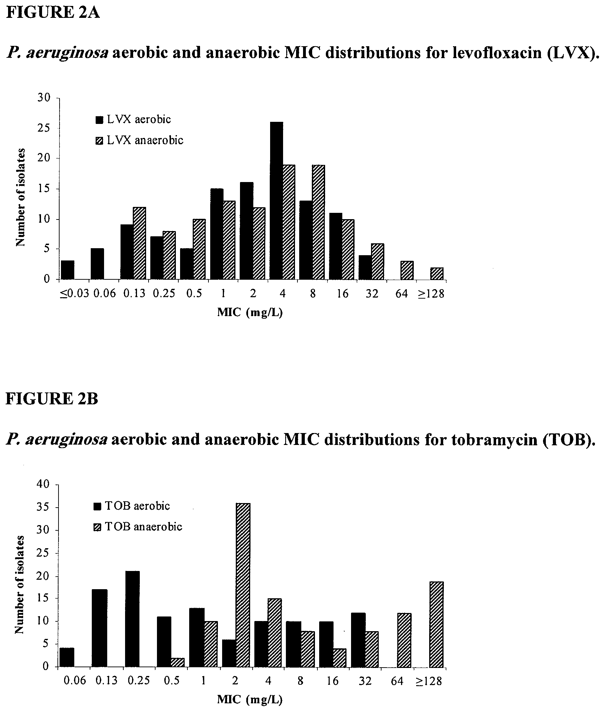

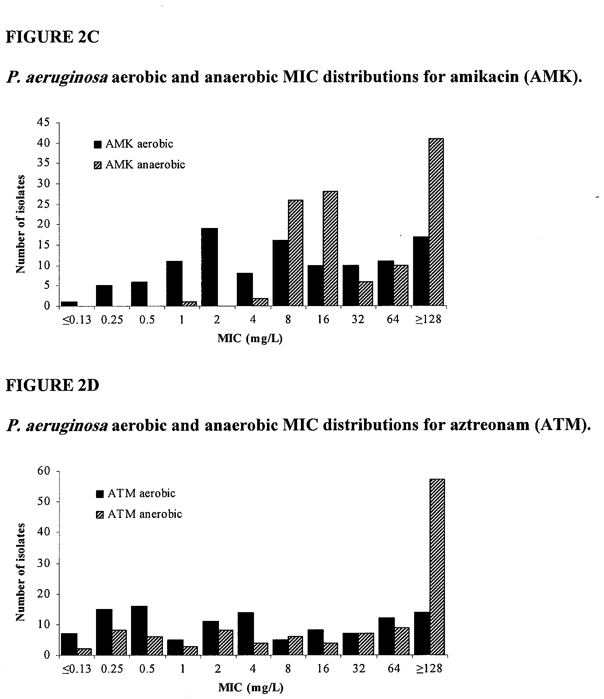

FIGS. 2A-D show the distribution of aerobic and anaerobic MIC results for each antibiotic with all 114 *P. aeruginosa* isolates. Under anaerobic conditions, tobramycin, amikacin, and aztreonam demonstrated reduced potency, indicated by the shift in MIC distribution. In contrast, the aerobic and anaerobic MIC distributions for levofloxacin were similar.

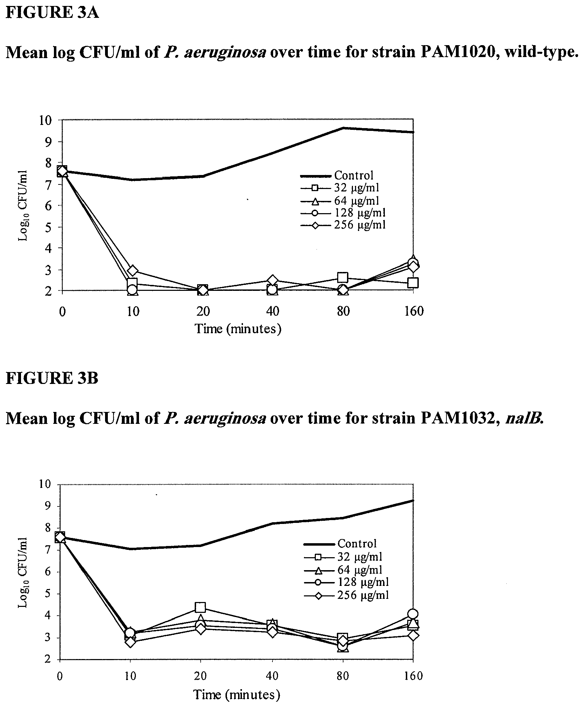

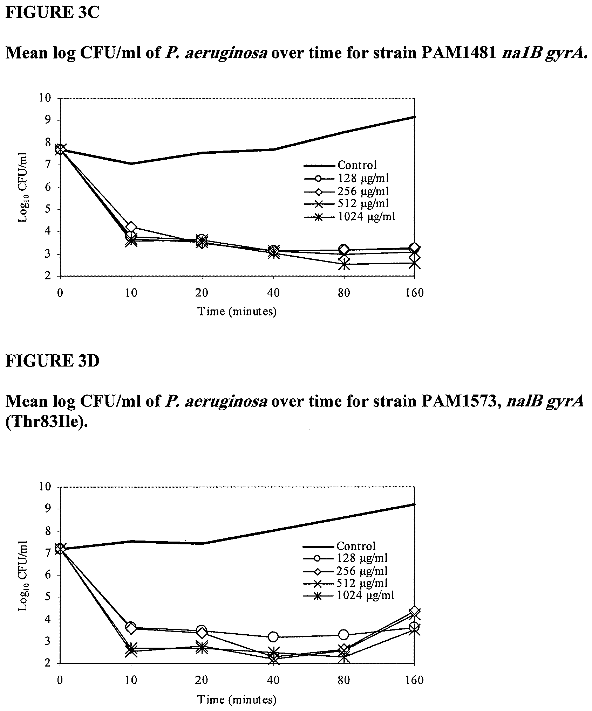

Time-kill curves were developed to determine the bactericidal activity of high concentrations of levofloxacin attained following aerosol administration against isogenic *P*. *aeruginosa* strains under aerobic and hypoxic conditions to simulate the partial oxygen gradient present in the lungs of CF patients. Rapid and sustained in vitro bactericidal activity within 10 minutes was observed for each strain at each levofloxacin concentration under both conditions (p>0.05), as shown in FIG. 3.

## Claims

1. A fluoroquinolone antibiotic for use in a method of treating a pulmonary bacterial infection, said method comprising administering a therapeutically effective amount of an aerosol of the fluoroquinolone antibiotic; wherein the fluoroquinolone antibiotic is levofloxacin, wherein the pulmonary bacterial infection comprises *Pseudomonas aeruginosa* bacteria growing under anaerobic conditions, and wherein the bacteria is exposed to at least 0.75 mg/L of the fluoroquinolone antibiotic.

2. The fluoroquinolone antibiotic for use of claim 1, said method further comprises before administering the fluoroquinolone antibiotic, assaying the pulmonary bacterial infection for the presence of bacteria growing under anaerobic conditions.

3. The fluoroquinolone antibiotic for use of claims 1 or 2, wherein the bacteria is capable of growing under anaerobic conditions using nitrate or nitrite.

4. The fluoroquinolone antibiotic for use of Claim 3, wherein said method further comprises before administering the fluoroquinolone antibiotic, assaying the pulmonary bacterial infection for the presence of bacteria capable of growing under anaerobic conditions using nitrate or nitrite.

5. The fluoroquinolone antibiotic for use of any one of claims 1 to 4, further comprising assaying the pulmonary bacterial infection for the presence of *Pseudomonas aeruginosa.*

6. The fluoroquinolone antibiotic for use of any one of claims 1 to 5, wherein said pulmonary bacterial infection is identified as having at least a portion of said bacteria growing under anaerobic conditions.

7. The fluoroquinolone antibiotic for use of any one of claims 1 to 6, wherein the pulmonary bacterial infection is in a subject with cystic fibrosis.

8. The fluoroquinolone antibiotic for use of any one of claims 1 to 7, wherein the pulmonary infection is **characterized by** sputum comprising nitrate levels of at least 250 µM.

9. The fluoroquinolone antibiotic for use of any one of claims 1 to 8, wherein said pulmonary bacterial infection is **characterized by** sputum comprising nitrate levels of at least 500 µM.

10. The fluoroquinolone antibiotic for use of any one of claims 1 to 9, wherein the method of treating the pulmonary bacterial infection does not include administering a therapeutically effective amount of an antibiotic selected from the group consisting of tobramycin, amikacin and aztreonam.

11. The fluoroquinolone antibiotic for use of any one of claims 1 to 10, wherein no other antibiotics are administered in a therapeutically effective amount to treat the pulmonary bacterial infection.

12. The fluoroquinolone antibiotic for use of any one of claims 1 to 11, wherein the fluoroquinolone antibiotic is administered by intrapulmonary delivery.

13. The fluoroquinolone antibiotic for use of any one of claims 1 to 12, wherein said therapeutically effective amount of fluoroquinolone is more than 5 mg.

14. The fluoroquinolone antibiotic for use of any one of claims 1 to 13, wherein said therapeutically effective amount of fluoroquinolone is no more than 150 mg.

15. A fluoroquinolone antibiotic for use in a method of inhibiting *Pseudomonas aeruginosa* bacteria growing under anaerobic conditions comprising exposing said bacteria to an effective amount of a fluoroquinolone antibiotic to inhibit the growth of said bacteria, wherein the bacteria is exposed to a mixture comprising at least 0.75 mg/L of the fluoroquinolone antibiotic; wherein the fluoroquinolone antibiotic is levofloxacin.

16. The fluoroquinolone antibiotic for use of claim 15, said further comprising assaying a sample of said bacteria to determine that said bacteria is growing under anaerobic conditions, before administering the fluoroquinolone antibiotic.

17. The fluoroquinolone antibiotic for use of any claim 15 or claim 16, wherein a sample of the bacteria is **characterized by** nitrate levels of at least 250 µM.

18. A fluoroquinolone antibiotic for use in a method of inhibiting *Pseudomonas aeruginosa* bacteria growing under anaerobic conditions comprising exposing said bacteria to an effective amount of a fluoroquinolone antibiotic to inhibit the growth of said bacteria, wherein the fluoroquinolone antibiotic is levofloxacin at a concentration ranging from 0.125 mg/L to 128 mg/L.

19. The fluoroquinolone antibiotic for use of any of claims 15 to 18, wherein the fluoroquinolone antibiotic is administered to a patient with a pulmonary bacterial infection caused by the *Pseudomonas aeruginosa* bacteria, for example wherein the fluoroquinolone antibiotic is administered to a cystic fibrosis patient with a pulmonary bacterial infection caused by the *Pseudomonas aeruginosa* bacteria.

## Patentansprüche

1. Fluorchinolon-Antibiotikum zur Verwendung in einem Verfahren zum Behandeln einer bakteriellen Lungeninfektion, wobei das Verfahren das Verabreichen einer therapeutisch wirksamen Menge eines Aerosols des Fluorchinolon-Antibiotikums umfasst; wobei das Fluorchinolon-Antibiotikum Levofloxacin ist, wobei die bakterielle Lungeninfektion *Pseudomonas aeruginosa*-Bakterien umfasst, die unter anaeroben Bedingungen wachsen, und wobei die Bakterien zumindest 0,75 mg/l des Fluorchinolon-Antibiotikums ausgesetzt werden.

2. Fluorchinolon-Antibiotikum zur Verwendung nach Anspruch 1, wobei das Verfahren ferner vor dem Verabreichen des Fluorchinolon-Antibiotikums das Untersuchen der bakteriellen Lungeninfektion auf das Vorhandensein von Bakterien umfasst, die unter anaeroben Bedingungen wachsen.

3. Fluorchinolon-Antibiotikum zur Verwendung nach Anspruch 1 oder 2, wobei die Bakterien dazu in der Lage sind, unter Verwendung von Nitrat oder Nitrit unter anaeroben Bedingungen zu wachsen.

4. Fluorchinolon-Antibiotikum zur Verwendung nach Anspruch 3, wobei das Verfahren ferner vor dem Verabreichen des Fluorchinolon-Antibiotikums das Untersuchen der bakteriellen Lungeninfektion auf das Vorhandensein von Bakterien umfasst, die dazu in der Lage sind, unter Verwendung von Nitrat oder Nitrit unter anaeroben Bedingungen zu wachsen.

5. Fluorchinolon-Antibiotikum zur Verwendung nach einem der Ansprüche 1 bis 4, ferner umfassend das Untersuchen der bakteriellen Lungeninfektion auf das Vorhandensein von *Pseudomonas aeruginosa.*

6. Fluorchinolon-Antibiotikum zur Verwendung nach einem der Ansprüche 1 bis 5, wobei die bakterielle Lungeninfektion als zumindest einen Teil der Bakterien, die unter anaeroben Bedingungen wachsen, aufweisend identifiziert ist.

7. Fluorchinolon-Antibiotikum zur Verwendung nach einem der Ansprüche 1 bis 6, wobei die bakterielle Lungeninfektion in einem Subjekt mit zystischer Fibrose ist.

8. Fluorchinolon-Antibiotikum zur Verwendung nach einem der Ansprüche 1 bis 7, wobei die Lungeninfektion durch Sputum gekennzeichnet ist, das Nitratwerte von zumindest 250 µM umfasst.

9. Fluorchinolon-Antibiotikum zur Verwendung nach einem der Ansprüche 1 bis 8, wobei die bakterielle Lungeninfektion durch Sputum gekennzeichnet ist, das Nitratwerte von zumindest 500 µM umfasst.

10. Fluorchinolon-Antibiotikum zur Verwendung nach einem der Ansprüche 1 bis 9, wobei das Verfahren zum Behandeln der bakteriellen Lungeninfektion nicht das Verabreichen einer therapeutisch wirksamen Menge eines Antibiotikums beinhaltet, das aus der Gruppe ausgewählt ist, die aus Tobramycin, Amikacin und Aztreonam besteht.

11. Fluorchinolon-Antibiotikum zur Verwendung nach einem der Ansprüche 1 bis 10, wobei keine anderen Antibiotika in einer therapeutisch wirksamen Menge verabreicht werden, um die bakterielle Lungeninfektion zu behandeln.

12. Fluorchinolon-Antibiotikum zur Verwendung nach einem der Ansprüche 1 bis 11, wobei das Fluorchinolon-Antibiotikum durch intrapulmonale Abgabe verabreicht wird.

13. Fluorchinolon-Antibiotikum zur Verwendung nach einem der Ansprüche 1 bis 12, wobei die therapeutisch wirksame Menge von Fluorchinolon mehr als 5 mg beträgt.

14. Fluorchinolon-Antibiotikum zur Verwendung nach einem der Ansprüche 1 bis 13, wobei die therapeutisch wirksame Menge von Fluorchinolon nicht mehr als 150 mg beträgt.

15. Fluorchinolon-Antibiotikum zur Verwendung in einem Verfahren zum Hemmen von *Pseudomonas aeruginosa-Bakterien,* die unter anaeroben Bedingungen wachsen, umfassend das Aussetzen der Bakterien gegenüber einer wirksamen Menge eines Fluorchinolon-Antibiotikums, um das Wachstum der Bakterien zu hemmen, wobei die Bakterien einem Gemisch ausgesetzt werden, das zumindest 0,75 mg/l des Fluorchinolon-Antibiotikums umfasst; wobei das Fluorchinolon-Antibiotikum Levofloxacin ist.

16. Fluorchinolon-Antibiotikum zur Verwendung nach Anspruch 15, ferner umfassend das Untersuchen einer Probe der Bakterien, um zu bestimmen, dass die Bakterien unter anaeroben Bedingungen wachsen, vor dem Verabreichen des Fluorchinolon-Antibiotikums.

17. Fluorchinolon-Antibiotikum zur Verwendung nach einem von Anspruch 15 oder Anspruch 16, wobei eine Probe der Bakterien durch Nitratwerte von zumindest 250 µM gekennzeichnet ist.

18. Fluorchinolon-Antibiotikum zur Verwendung in einem Verfahren zum Hemmen von *Pseudomonas aeruginosa*-Bakterien, die unter anaeroben Bedingungen wachsen, umfassend das Aussetzen der Bakterien gegenüber einer wirksamen Menge eines Fluorchinolon-Antibiotikums, um das Wachstum der Bakterien zu hemmen, wobei das Fluorchinolon-Antibiotikum Levofloxacin in einer Konzentration ist, die von 0,125 mg/l bis 128 mg/l reicht.

19. Fluorchinolon-Antibiotikum zur Verwendung nach einem der Ansprüche 15 bis 18, wobei das Fluorchinolon-Antibiotikum einem Patienten mit einer bakteriellen Lungeninfektion verabreicht wird, die durch die *Pseudomonas aeruginosa*-Bakterien verursacht wird, wobei das Fluorchinolon-Antibiotikum zum Beispiel einem Patienten mit zystischer Fibrose mit einer bakteriellen Lungeninfektion verabreicht wird, die durch die *Pseudomonas aeruginosa-Bakterien* verursacht wird.

## Revendications

1. Antibiotique de fluoroquinolone destiné à être utilisé dans un procédé de traitement d'une infection bactérienne pulmonaire, ledit procédé comprenant l'administration d'une quantité thérapeutiquement efficace d'un aérosol de l'anticorps de fluoroquinolone ; dans lequel l'anticorps de fluoroquinolone est la lévofloxacine, dans lequel l'infection bactérienne pulmonaire comprend des bactéries *Pseudomonas aeruginosa* se développant dans des conditions d'anaérobie, et dans lequel les bactéries sont exposées à au moins 0,75 mg/L de l'antibiotique de fluoroquinolone.

2. Antibiotique de fluoroquinolone destiné à être utilisé selon la revendication 1, ledit procédé comprend en outre avant l'administration de l'antibiotique de fluoroquinolone, le dosage de l'infection bactérienne pulmonaire pour révéler la présence de bactéries se développant dans des conditions d'anaérobie.

3. Antibiotique de fluoroquinolone destiné à être utilisé selon les revendications 1 ou 2, dans lequel les bactéries sont capables de se développer dans des conditions d'anaérobie en utilisant du nitrate ou du nitrite.

4. Antibiotique de fluoroquinolone destiné à être utilisé selon la revendication 3, dans lequel ledit procédé comprend en outre avant l'administration de l'antibiotique de fluoroquinolone, le dosage de l'infection bactérienne pulmonaire pour révéler la présence de bactéries capables de se développer dans des conditions d'anaérobie en utilisant du nitrate ou du nitrite.

5. Antibiotique de fluoroquinolone destiné à être utilisé selon l'une quelconque des revendications 1 à 4, comprenant en outre le dosage de l'infection bactérienne pulmonaire pour révéler la présence de *Pseudomonas aeruginosa.*

6. Antibiotique de fluoroquinolone destiné à être utilisé selon l'une quelconque des revendications 1 à 5, dans lequel ladite infection bactérienne pulmonaire est identifiée comme ayant au moins une partie desdites bactéries se développant dans des conditions d'anaérobie.

7. Antibiotique de fluoroquinolone destiné à être utilisé selon l'une quelconque des revendications 1 à 6, dans lequel ladite infection bactérienne pulmonaire est chez un sujet atteint de mucoviscidose.

8. Antibiotique de fluoroquinolone destiné à être utilisé selon l'une quelconque des revendications 1 à 7, dans lequel l'infection pulmonaire est **caractérisée par** une expectoration comprenant des niveaux de nitrate d'au moins 250 µM.

9. Antibiotique de fluoroquinolone destiné à être utilisé selon l'une quelconque des revendications 1 à 8, dans lequel ladite infection bactérienne pulmonaire est **caractérisée par** une expectoration comprenant des niveaux de nitrate d'au moins 500 µM.

10. Antibiotique de fluoroquinolone destiné à être utilisé selon l'une quelconque des revendications 1 à 9, dans lequel le procédé de traitement de l'infection bactérienne pulmonaire n'inclut pas l'administration d'une quantité efficace thérapeutiquement d'un antibiotique sélectionné parmi le groupe constitué par la tobramycine, l'amikacine et l'aztréonam.

11. Antibiotique de fluoroquinolone destiné à être utilisé selon l'une quelconque des revendications 1 à 10, dans lequel aucun autre antibiotique n'est administré dans une quantité thérapeutiquement efficace pour traiter l'infection bactérienne pulmonaire.

12. Antibiotique de fluoroquinolone destiné à être utilisé selon l'une quelconque des revendications 1 à 11, dans lequel l'antibiotique de fluoroquinolone est administré par délivrance par voie intrapulmonaire.

13. Antibiotique de fluoroquinolone destiné à être utilisé selon l'une quelconque des revendications 1 à 12, dans lequel ladite quantité thérapeutiquement efficace de fluoroquinolone est supérieure à 5 mg.

14. Antibiotique de fluoroquinolone destiné à être utilisé selon l'une quelconque des revendications 1 à 13, dans lequel ladite quantité thérapeutiquement efficace de fluoroquinolone n'est pas supérieure à 150 mg.

15. Antibiotique de fluoroquinolone destiné à être utilisé dans un procédé d'inhibition des bactéries *Pseudomonas aeruginosa* se développant dans des conditions d'anaérobie comprenant l'exposition desdites bactéries à une quantité efficace d'un antibiotique de fluoroquinolone pour inhiber le développement desdites bactéries, dans lequel les bactéries sont exposées à un mélange comprenant au moins 0,75 mg/L de l'antibiotique de fluoroquinolone ; dans lequel l'antibiotique de fluoroquinolone est la lévofloxacine.

16. Antibiotique de fluoroquinolone destiné à être utilisé selon la revendication 15, comprenant en outre le dosage d'un échantillon desdites bactéries pour déterminer que lesdites bactéries se développent dans des conditions d'anaérobie, avant l'administration de l'antibiotique de fluoroquinolone.

17. Antibiotique de fluoroquinolone destiné à être utilisé selon la revendication 15 ou la revendication 16, dans lequel un échantillon des bactéries est **caractérisé par** des niveaux de nitrate d'au moins 250 µM.

18. Antibiotique de fluoroquinolone destiné à être utilisé dans un procédé d'inhibition des bactéries *Pseudomonas aeruginosa* se développant dans des conditions d'anaérobie comprenant l'exposition desdites bactéries à une quantité efficace d'un antibiotique de fluoroquinolone pour inhiber le développement desdites bactéries, dans lequel l'antibiotique de fluoroquinolone est la lévofloxacine à une concentration comprise dans la plage de 0,125 mg/L à 128 mg/L.

19. L'antibiotique de fluoroquinolone destiné à être utilisé selon l'une quelconque des revendications 15 à 18, dans lequel l'antibiotique de fluoroquinolone est administré à un patient avec une infection bactérienne pulmonaire causée par les bactéries *Pseudomonas aeruginosa,* par exemple, dans lequel l'antibiotique de fluoroquinolone est administré à un patient atteint de mucoviscidose avec une infection bactérienne pulmonaire causée par les bactéries *Pseudomonas aeruginosa.*
